Europäisches Patentamt

European Patent Office

Office européen des brevets

(11) Publication number: **0 279 097**

**A2**

## EUROPEAN PATENT APPLICATION

(21) Application number: 87303879.8

(51) Int. Cl.4 **G01N 33/543** , G01N 33/52

(22) Date of filing: 30.04.87

(30) Priority: 17.02.87 US 14985

(43) Date of publication of application:
24.08.88 Bulletin 88/34

(84) Designated Contracting States:
BE DE ES FR GB IT LU NL SE

(71) Applicant: **GENESIS LABS, INC.**
**5182 W. 76th St.**
**Edina Minnesota 55435(US)**

(72) Inventor: **Fuerstenberg, Richard K.**
**1233 Galtier Street**
**St.Paul Minnesota 55117(US)**

(74) Representative: **Bond, Bentley George et al**
**HASELTINE LAKE & CO. Hazlitt House 28**
**Southampton Buildings Chancery Lane**
**London, WC2A 1AT(GB)**

(54) Immunoassay test strip.

(57) An immunoassay dry test strip comprising a matrix having three zones wherein bypass of the second zone of test fluid containing analyte and label from the first zone is prevented by means to seal the second zone. The first zone contains a labeled-antibody composition having at least one specific analyte-antigen binding site; the second zone contains immobilized antigen composition that can bind specifically with an analyte-antigen binding site on said labeled-antibody; and the third zone, removed from said first and second zones, contains detection means for indicating the presence of the label of said labeled-antibody within said third zone.

FIG. 1

## IMMUNOASSAY TEST STRIP

Field of the Invention

The invention relates to an immunoassay dry test strip and a method of using the test strip for determining the presence of an analyte-antigen or an analyte-antibody or a fragment thereof.

Background of the Invention

Diagnostic tests in many formats employing specific immunological antigen-antibody binding reactions are used for determining the presence of antibodies or antigens of many kinds in biological and other fluids. The test formats include wet chemical methods and dry test strips. Dry test strips can be used to detect an analyte by applying a test fluid and other reactive solutions to generate a unique signal. Signals can be generated by many means including enzyme reagents, fluorescent markers, radioactive particles and others.

The Enzyme Linked Immunosorbent Assay (ELISA), or Enzyme Immunoassay (EIA) techniques employ an enzyme label which is used as a detection marker. Differing EIA formats have been developed, but all employ an enzyme label, detection means for determining the presence of the label, and a specific immunological binding reaction of an antigen-antibody pair. Although these assays can be somewhat simpler for laboratory technicians relative to other means for detecting the presence of an analyte species, these assays can be lengthy and can involve several steps and reagents.

One recently developed EIA method, disclosed in Liotta, U.S. Pat. No. 4,446,232, is a singularly unique response to the need for a one-step testing procedure providing a rapid, reliable result. The study of the Liotta technology in the laboratory has generated new ideas for ways to apply such a test strip for determining the presences of an analyte. We have found that the rapid, one-step, accurate Liotta technology and test strip performance can be further enhanced by reducing test strip exposure to environmental variables, such as atmospheric humidity and air flow, during testing. Further, consideration has been given to providing a means to insure that the test fluid is applied and analyzed accurately and that the volume of the test fluid sample is controlled. Given the desire to mass produce such a test strip for use in the field of diagnostic testing, any embodiment of the Liotta technology should be useful in a manufacturing context.

Summary of the Invention

The present invention provides an immunoassay test strip for determining the presence of an analyte-antigen or an analyte-antibody or fragments thereof, a the method of using this test strip. The test strip of the invention comprises a Liotta-type device having a matrix of three zones, a first labeled reagent zone, a second trapping zone having bound antigen or antibody, and a third detection zone for label detection, each zone held within an enclosure. The test strip further comprises sealing means-to prevent test fluid bypass of the second zone to the third zone of test fluid applied to the first zone. Any bypass that occurs could result in a false signal in the detection zone caused by labeled-reagent passing to the third zone outside of the second zone. The test fluid, as a result of the presence of the sealing means, passes from the first zone through the second zone into the third zone, resulting in the appropriate detection of an analyte if present.

In one aspect, the invention is directed to a test device for determining the presence of an analyte-antigen. The test device comprises a matrix within an enclosure, said enclosure having a first opening for application of a test fluid containing an analyte-antigen. The matrix comprises a first zone containing a labeled-antibody, or fragment thereof, which has at least one specific analyte-antigen immunological binding site, a second zone containing immobilized antigen, which can bind specifically with the analyte-antigen binding site on the labeled-antibody, and a third zone, removed from the first and second zones, containing detection means for indicating the presence of the antibody label within said third zone. In the context of this invention, in the zone, an antibody composition can be replaced with an active binding antibody fragment such as an Fab or $F(ab')_2$.

A test fluid containing an analyte-antigen that can specifically bind the labeled-antibody occupying the specific binding site of the labeled-antibody, will cause the labeled-antibody to pass from the first zone, through the second zone, to stimulate an appropriate detection means response in the third zone. If no analyte-antigen is present in the test fluid to bind specifically with the analyte-antigen binding site on the labeled-antibody, the unbound labeled-antibody will bind to immobilized antigen in the second zone, restricting or trapping the labeled-antibody to the second zone and thereby preventing it from passing through the second zone into the third zone preventing signal generation and detection. Detection can be quantitative and can be in direct proportion to the concentration of the antigen in the test fluid. In the total absence of antigen-analyte, the detection means will not be stimulated and will not respond, indicating the absence of the labeled-antibody in the third zone, and the absence of analyte-antigen in the test fluids.

The enclosure of the present invention limits exposure of the test strip to variable environmental parameters such as atmospheric humidity and air flow and further insures proper fluid flow within the test strip. In implementing these parameters, the potential variation in test conditions can be controlled, and the potential variation in test results can be reduced. This is particularly important in adapting the test to provide a quantitive measure of analyte concentration.

The enclosure has a first opening for locating a uniform application of the test fluid on the test strip. Additional openings can be incorporated in the enclosure to allow air to escape when displaced by the passage of the test fluid through the matrix. This facilitates a uniform test fluid diffusion through the matrix and uniform detection. The enclosure has further utility in another respect. The enclosure acts to provide control on the volume of sample which the test device will accept. Given the volume capacity of the enclosure, the aliquot of test fluid applied is necessarily limited to this volume in a proper use of the device.

In accord with a preferred embodiment of the present invention, the means for preventing bypass of the second zone by test fluid conveying labeled-antibody from the first zone to the third zone, comprises an arrangement of the first zone, the second zone and the third zone such that fluid communication between the first zone and the third zone is exclusively provided by the second zone. In more preferred embodiments fluid communication is limited to the second zone by the enclosure sealingly engaging at least the second zone to cause fluid to pass through the second zone to eliminate bypass of second zone by test fluid conveying labeled-antibody.

In the most preferred embodiments the peripheral edge of the second zone is sealingly engaged to a polymeric carrier strip. The first and third zones are brought into fluid contact with the second zone are covered by a clear sealing means which completes the enclosure assembly. The enclosure interior surface can in certain instances provide an avenue for fluid communication from the first zone to the third zone if some means of preventing this communication is omitted. Such a transmission on the interior surface of the enclosure can result in false positive results. By sealing the peripheral edge of the second zone to the enclosure, i.e. either the polymeric carrier strip or the clear plastic film sealing means, the test fluid is forced to flow through the second zone to the third zone as the test device function requires.

In a second aspect, the present invention is directed to a method of using the test device described above for determining the presence of an analyte-antigen by applying a test fluid to the strip.

In a third aspect, the present invention is directed to a test device for determining the presence of an analyte-antibody or a fragment thereof. This device comprises the same elements as the test device described above, except that the device detects the presence of analyte-antibody, uses a labeled antigen in the labeled reagent layer and a bound antibody in the trapping layer.

In a fourth aspect, the present invention is directed to a method of using the test device for determining the presence of the analyte-antibody.

Brief Description of Drawings

FIGURES 1, 2 and 3 illustrate the embodiment of the dry-test strip device of the invention configured for the detection of an antigen-analyte. Figs. 1-3 further show the preferred construction of the zones of the test strip and a preferred mode of sealing the edge of the second zone to prevent bypass.

FIGURE 4 is a cross-sectional diagram of a preferred embodiment of the test strip of the invention showing the interrelationship between the carrier strip, the zones of the test strip device and means to seal the device.

FIGURE 5 is an exploded diagram showing the relationship between the carrier strip, the sealing means and the zones.

## Detailed Description of the Invention

Briefly, the invention involves a test strip device comprising a first or labeled reagent zone, a second or binding, trapping zone, and a third or detection zone. In a device for the detection of an antigen, the labeled reagent zone contains labeled antibody and the binding zone contains immobilized antigen. In a device for the detection of antibody the reagent zone contains labeled antigen and the trapping zone contains immobilized antibody. The detection means in the detection zone detects the presence of a label and is typically not changed between an analyte-antigen or an analyte-antibody detecting device.

Each zone of the Liotta matrix can comprises a sheet-like layer having a peripheral edge. The second zone is positioned between the first zone and the third zone and the edge of the second zone layer can outwardly extend past the edge of the first and third zones. In the most preferred embodiment, the peripheral edge of the second zone layer extending past the edge of the third zone can be sealingly engaged to the enclosure to eliminate bypass of test fluid conveying labeled-antibody. Each zone is typically less than 2 cm in major dimension. In the preferred circular zones the diameter is preferably less than 1.5 cm and can range from 0.25 to 1.25 cm. In the preferred mode the second zone has a diameter greater than the first and third zone by 0.4 to 1 cm.

The technology of the present invention is applicable to the detection of antigens and antibodies. For detecting antigen, the antibody is labeled and its antibody-antigen pair partner is immobilized and contained within the second zone. In an antigen detective strip, the labeled antibody will bind to the immobilized antigen in the second zone in the absence of analyte-antigen in the test fluid, and will be prevented from diffusing into the third zone where it would otherwise stimulate a response from the detection means.

In an antibody detection strip, antigen, present in the test fluid, will bind to the labeled antibody. Because some antibody (e.g., IgM) can be large, more than one antigen can bind simultaneously. This embodiment works best with small antibody or an antibody fragment whose size is such that when a single antigen is bound to the labeled antibody, further binding of other specific antigen-antibody pair partners can be prevented by steric hindrance caused by the bound antigen or because a single binding site exists. For detecting antibodies, the antigen is labeled and its antigen-antibody pair partner is immobilized and contained within the second zone. The labeled-antigen will bind to the immobilized antibody in the second zone in the absence of analyte-antibody in the test fluid, and will be prevented from diffusing into the third zone where it would otherwise stimulate a response from the detection means. If antibody is present in the test fluid, they will bind to the labeled-antigen. Because some antigens are so large that more than one antibody may bind to them at one time, this embodiment works best with small antigens whose size is such that when a single antibody is bound to the labeled antigen, further binding of other specific antigen-antibody pair partners can. be prevented by steric hindrance caused by the antibody already binding the antigen or because only a single epitope or antigenic binding site exists.

In an alternate embodiment, the test seeks to determine the presence of antibodies of large antigens. In this embodiment, the antigens can be broken into small fragments which correspond to each of the epitopes that can elicit an immune response. Preferably each fragment can be capable of binding only a single antibody and must effectively bind the antibody such that diminished specificity will not allow the antibodies to disassociate from the antigen fragment in the presence of additional specific competing antigen-antibody pair partners.

### Analyte

A typical analyte-antigen detected by the device of this invention is characterized by being a monoepitopic or polyepitopic. Polyepitopic analytes are typically large molecule polypeptides, polysaccharides, polynucleic acids, and combinations thereof. Other analytes can be somatic cells, germ cells, bacteria, viruses, and other cellular units. Sub-cellular units which can be analytes include viral coat protein, cell wall polysaccharide, DNA, DNA segments, transfer RNA, messenger RNA, mitochondrial DNA, mitochondria, cell nuclei, cell membrane, ribosome, and other varied cell organelles, sub-units and constituent parts. Such polyepitopic analytes detected by the invention typically can have molecular weights in excess of about 50,000. Many such analytes can have a molecular weight ranging from 50,000 to 5,000,000 and more.

The analytical device of this invention can also be used to detect and quantitate the presence of smaller molecular analyte-antigens (i.e. molecules with a molecular weight less than about 50,000), typically between 5,000 and 50,000. The device of this invention can be used to analyze for the presence of virtually any analyte that can elicit an antibody response. The analyte can be either the antigen or an epitope containing fragment thereof.

A wide variety of natural proteins and protein sub-units can be detected using the device of the invention. Such proteins include histones, globulins, nucleoproteins, lipoproteins, glycoproteins, somatotropin, prolactin, insulin, pepsin, human plasma protein constituents including human albumen, thyroxin binding globulin, haptoglobin, ceruloplasmin, cholinesterase, myoglobin, fibrinogen, plasminogen, poly-and monoclonal immunoglobulins of the A, D, E, G or M classes, free light or heavy chains of the immunoglobulins, an Fab fragment or an F(ab')₂ fragment, variable regions, hypervariable regions, or constant regions of the immunoglobulin; complement factors, bloodclotting factors such as thromboplastin (factor III), Christmas factor (factor IX), and others; peptide and protein hormones including glucagon, erythropoietin, FSH, LH, kinetotropins including HCG, oxytocin, vasopresin. Clearly, any protein or protein sub-unit that can generate an antibody in poly or monoclonal antibody techniques can be used in the invention.

Antigenic polysaccharides derived typically from cell walls of pathogens (such as virus, bacteria, yeasts, fungi) can act as antigen. Such cell wall antigens can be detected in strains of microorganisms such as Strepylococcus, Corynebacterium, Haemophilus, Klebsiella, Salmonella, Candida yeasts, Actinomycetes fungi, pneumococcus, Streptococcus, Staphylococcus, Anthrobacteria, and others.

Small molecules of natural and synthetic origin, typically monoepitopic analytes having a molecular weight of about 100 to 5,000, typically 100 to 2,000 can also act as an analyte. Such analytes include small molecule natural biochemicals; ethical, over the counter, and illicit drugs, hormones, peptides, mono and disaccharides, metabolites, pesticides, pollutants, and other organic synthetic chemicals, etc. Drugs of interest include alkaloids such as morphine, codeine, heroin, dextromethorphan, derivatives and metabolites. Also included are ergot alkaloids such as LSD, steroid alkaloids, quinoline alkaloids and others. Ethical drugs of interest include steroids, bile acids, digoxin, diethylstilbestrol, ethynylestradiol, and others. Other drugs include barbiturates such as phenolbarbitol, secobarbitol, and others. Additionally, drugs such as amphetamines, catechol amines, L-dopa, epinephrin, chlorpromazine, benzodiazipines, phenolthiazines, theophylline, caffeine, canibis drugs such as cannabinol, tetrahydrocannabinol, vitamins, prostaglandins, antibiotics, such as penicillin, and the penicillin variants, cephalosporin and the cephalosporin variants, chloromycetin, actinomycetin, tetracycline, nucleosides and nucleotides, fragments and derivatives thereof including ATP, NAD, FMN, AZTP, and others. Additionally drugs including methadone, meprobamate, serotonin lidocaine, propranolol, antihistamines, anticholinergic drugs, and others can be detected.

Antibody useful in preparing the dry test strip devices of this invention can be prepared in well known polyclonal and monoclonal antibody techniques. Polyclonal antibodies can be raised in a variety of test animals including mice, rats, rabbits, horses, and others. Monoclonal antibodies can be prepared using well known techniques such as that disclosed by Kohler and Milstein, "Continuous Cultures of Fused Cells Secreting Antibody of Predetermining Specificity", Nature, Vol. 256, pp. 495-497, August 7, 1975.


Zone Substrate Materials

The labeled reagent including labeled antigen, labeled antibody or labeled fragment thereof, the bound antigen or bound antibody and the signal generating zone can be formed in a wide variety of substrate materials. The primary function of the substrate materials is to act as a site or locus for the individual materials which permits the effective flow of the test fluid through the device to permit the reaction between antigen and antibody and to permit the signal generation mechanism to operate. The substrate can be of a variety of shape and form having varied dimensions depending on the applicability of the material in production. The support typically will have a thickness of at least 0.1 micron, typically greater than 1 micron, generally in the range of 10 to 100 microns. Substrates can be opaque, translucent or transparent, however the signal generated by the appropriate detection of the label present in the test device should not be masked by the nature of the support nor interfered with by constituents of the test fluid which may be removed at the application site opposite that of the signal generating zone.

Various materials can be employed as the substrate composition designed to avoid interfering in signal generation, passage of the test fluid, and reaction of the components. A wide variety of organic and inorganic polymers both natural and synthetic may be employed in each zone including polyethylene, polyvinyl chloride, polypropylene, poly-4-methylbutene, polystyrene, polymethacrylate, polyethylene terephthalate, rayon, nylon, polyvinyl butyrate, silicone films, polyformaldehyde, cellulose, cellulose acetate, nitrocellulose, etc. Other materials which may be considered include paper, glass, fiberglass, ceramics, metals, metal foils, metalloids, semi-conductive materials, and others. Additionally natural substances that can form gels or films including proteins or protein derivatives, cellulosics, drying oils, and others. The support material is preferably nonswellable, and mildly hydrophilic.

Label

In the case of Enzyme Linked Immunosorbent Assays (ELISA) or Enzyme Immunoassays (EIA), the label is an enzyme whose presence may be detected when it reacts with other constituents to give a detectable reaction product. Fluorescent Antibody Assays often employ similar immunoassay formats, but use a label which is fluorescent and whose presence may be directly detected. Radioimmunoassays (RIA) use a label which emits radioactive particles which are capable of detection. The present invention is not restricted to a specific means of detection or a specific type of label. In preferred embodiments, however, the detection means employs an enzyme label and may, therefore, be properly called an ELISA or an EIA. Some of the enzymes which can be used include acetyl cholinesterase, cytochrome C, beta-D-galactosidase, glycoamylase, glucose oxidase, beta-D-glucuronidase, lactate dehydrogenase, lactoperoxidase, ribonuclease, tyrosinase, alkaline phosphatase and horseradish peroxidase. Horseradish peroxidase, glucose oxidase and alkaline phosphatase are the enzymes of choice in preferred embodiments.

The preparation of a labeled reagent for the reagent zone typically involves conjugating the label composition to the antibody or antigen molecule. With many labels, for example enzymes, fluorescing labels, and others the label is often commercially available with an active group useful in protein labeling. Often the active group involves an activated carbonyl including nitrogen and sulfur analogs, illustrated by carboxylic acids activated with carbodiimide, carbonate monoester, in a mixed anhydride, carbonyl chloride, active ester, for example n-hydroxy succinamide, paranitrophenyl isocyanate, isothiocyanate, imidate ester, thioester, thionoester, and others. Reductive alkylation, active halogenation, and other well known techniques can be used. Often the labeled antigen or labeled antibody is available on the market. In one method of preparation of an antibody peroxidase conjugate, the peroxidase molecule is activated by treatment with sodium meta periodate. This treatment causes the saccharide units of the carbohydrate portion or portions of the peroxidase molecule to be oxidized, thus forming aldehyde groups. These aldehydes couple to primary amino groups in a Schiff base reaction that is subsequently stabilized by addition of sodium borohydride. The resulting conjugate mixture is then separated from unreacted components by one of a variety of methods.

The present invention, and immunoassays in general, depend upon the immunological recognition and binding reaction which is characteristic between a specific antigen-antibody pair. In the context of this invention, an antigen-antibody pair refers to an antigen (meaning any molecular species which elicits an immune response resulting in the production of antibodies which bind specifically to said molecular species) and its specific antibody (meaning antibody or a fragment thereof, produced in response to the antigen, which has at least one recognition site characteristically capable of a specific immunological recognition and binding reaction with the specific antigen). An antigen-antibody pair partner is the corresponding antigen or antibody with which an antigen or antibody has a specific antigen-antibody pair relationship with. This phenomenon allows either the antibody or the antigen to recognize and bind to its antigen-antibody pair partner practically irrespective of what other competing molecules may be in solution with the antigen-antibody pair partners. Each antigen may have a plurality of specific antibodies which can recognize and bind the antigen either at the same or at different locations. In addition, each antibody, while having a specific antigen which it recognizes and binds with specificity, may interact in a similar way with other antigens which present the same or similar molecular structural characteristics of the specific antigen recognition site or epitope which the antibody characteristically recognizes and binds. Such interactions are quite rare, given the idiosyncratic nature of naturally occurring molecular species, and do not typically interfere with antigen-antibody pair partner recognition and binding.

The immunoassay format which is employed by the present invention comprises an enclosed porous matrix having three zones. The enclosure has at least a first opening for application of a test fluid. Each zone in the matrix may be contiguous with one another or they may be removed from one another. It is possible for the first and second zones to occupy the same physical space and the same matrix. An essential feature is that the zones are porous and that the zones can be brought into fluid communication such that fluid can pass or communicate from one zone to another.

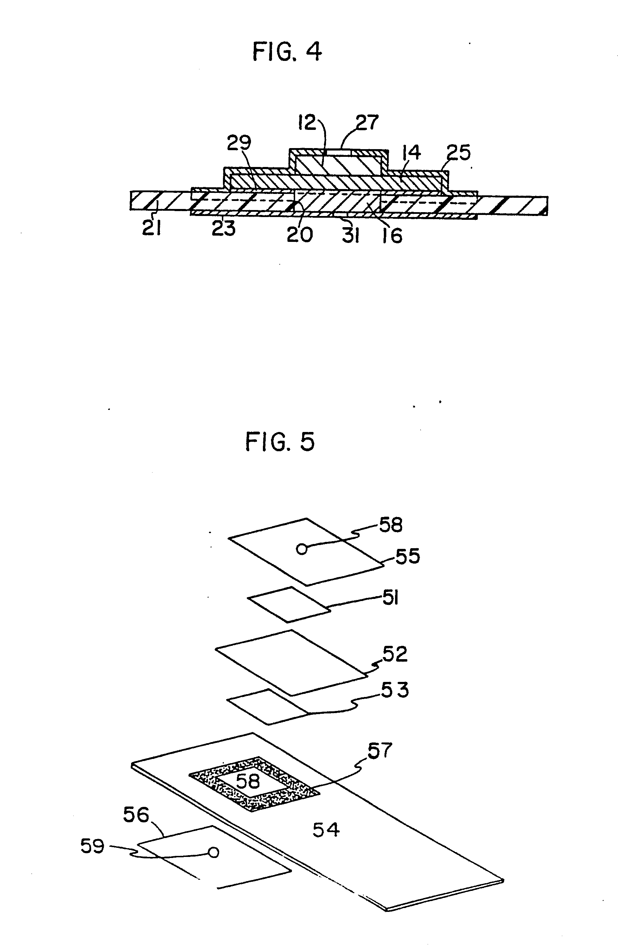

Another essential feature is that the fluid may not pass from the first zone to the third zone without passing through the second zone. The respective zones may be of any shape compatible with practical use of the invention. They may be square, rectangular or round, so long as the fluid is prevented from bypassing the second zone. In preferred embodiments, the second zone is larger than the third zone. In a more preferred embodiment, the peripheral edge of the second zone is sealingly engaged to the enclosure such that the test fluid is prevented from bypassing the second zone when conveying the labeled antigen-antibody pair partner from the first zone to the third zone. The second zone can be bonded to the enclosure in any suitable fashion. It is necessary to seal the entire peripheral edge of the second zone to the enclosure in this embodiment to prevent the fluid from bypassing the second zone by running along the surface of the enclosure. In the most preferred embodiment shown in Fig. 4, the enclosure incorporates a plastic carrier to which the second zone is sealed with an adhesive layer such as a pressure sensitive adhesive or a two-sided adhesive strip. The entire peripheral edge of the second zone extends outwardly beyond the edge of the third zone and is supported by the carrier to which it is sealingly engaged by the two-sided adhesive such that the test fluid must penetrate the second zone in passing from the first zone to the third zone. The second zone matrix is sealingly engaged to the strip with a double adhesive material such as two-sided adhesive tape. In the most preferred embodiment, doubly adhesive coated tape was used (3M #49485; 3M Corp., St. Paul, MN).

The fluid communication format is designed to allow test fluid which is applied to an opening or sample aperture in the first zone to pass or diffuse through the first zone, into the second zone, and through the second zone, into the third zone. This diffusion or passage of fluid through the matrix may occur in any manner conceivable. It may be urged by atmospheric pressure, gravitational forces, capillary action, surface action, osmotic forces, and the like. Although the applicant does not wish to be held to a theory of action, in a preferred embodiment the inclusion of a second opening in the matrix enclosure to allow displaced air to escape, may encourage the passage of fluid through the enclosed matrix by allowing the air in the matrix to be easily displaced, thereby removing any resistance to gravitational forces urging the fluid to diffuse through the matrix in a downward direction. In this case, the zones are arranged so that the downward gravitational diffusion of the test fluid will carry the fluid through the first zone, into and through the second zone, and subsequently, into the third zone. It may be that this format allows capillary action or other forces to urge the diffusion of fluid through the matrix. It has been noted, however, that this format allows a faster diffusion through the matrix than would otherwise occur. This rapid diffusion speeds the detection means response, thereby reducing the length of the incubation period and the overall time required to receive the test results.

The dimensions of the opening or sample aperture in the matrix enclosure is less than 10 millimeters, is preferably about 1 to 5 millimeters, and most commonly is between 2 and 5 millimeters in diameter. The diameter of the aperture permitting escape of air displaced by the transmittal of the test fluid through the matrix, is typically equal to or less than the diameter of the sample aperture.

The test strips described above can be used in a method to detect the presence of analyte-antigen or antibody-antigen in virtually any test fluid including fluids from biological sources that can be applied to the dry test strip. Fluids that can be applied to the dry test strip include urine, whole blood, blood components such as blood serum, blood plasma, etc.; cerebral spinal fluid, and other biological fluid extracted from human and animal body cavities. Further, analytes derived from tissue or other solid sources can be determined if the tissue or other solid substances homogenized and extracted with compatible liquids to contain an antigen-analyte or antigen-antibody arising from the solid tissue source.

Typically a small amount of the fluid or biological fluid is applied to the sample aperture on the dry test strip. The sample volume applied is typically measured in microliter amounts and can range from about 1 to 500 microliters depending on the analyte in the dry test strip internal volume. The diffusion of the test fluid through the dry test strip occurs in 1 to 5 minutes and a detectable signal can be generated within that time frame. The test fluid can be applied to the dry test strip using any means that can support a drop or drops of the test fluid including pipettes, micropipettes, stirring rods, wooden splints, and others. A substantial benefit in reproducibility can be obtained by applying the test fluid in repeatable volumes using a micropipette system.

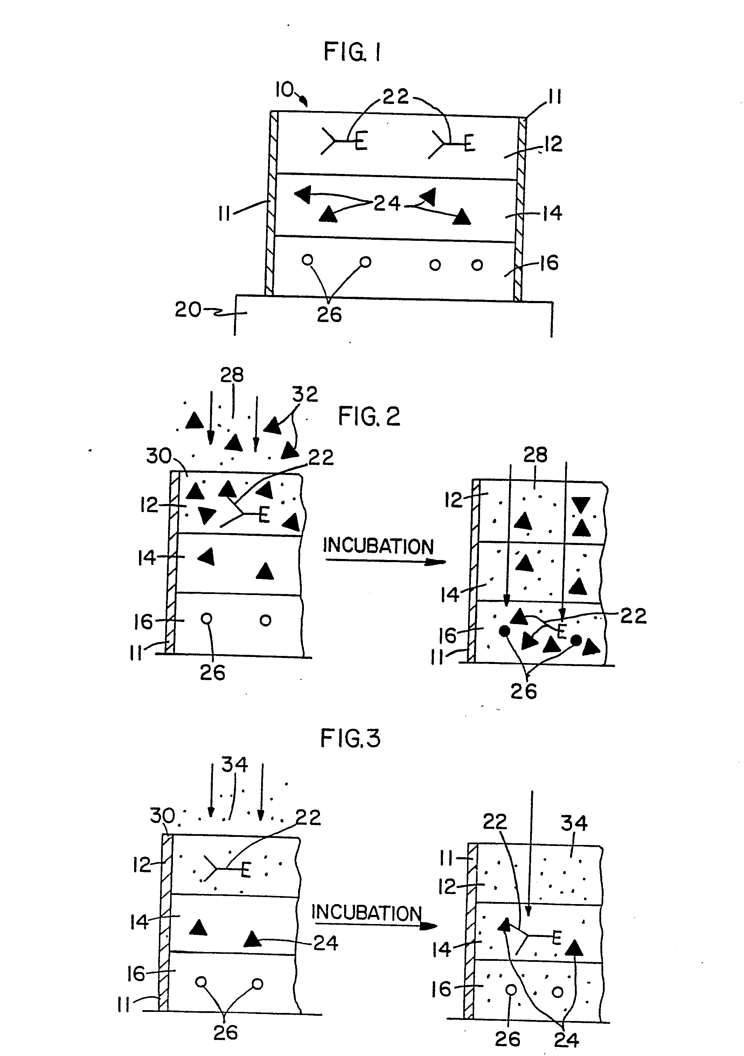

The zones of the enclosed matrix of the test device are impregnated with the assay reagents. In the preferred embodiment each zone is independent of the others and comprises a layer in a "sandwich" relationship wherein the zones are laminated one on top of the other such that one is on two and two is on three as shown in Fig. 1.

The first zone is impregnated with labeled-antibody or labeled-antigen. When aqueous test fluid is applied to the first zone the labeled entity is solubilized in the test fluid and may be conveyed out of the first zone by said fluid as it diffuses out of the first zone. The free reagents may be attached to the matrix by dissolving the assay reagents in fluids which are brought into contact with the matrix. The fluids may pass through the matrix leaving the reagents behind to dry or the fluid may be frozen in and around the matrix material of the zone, after which the water is evaporated through a freeze drying or lyophilizing process leaving behind the dry assay reagents. These and other methods of impregnating the matrix with the test reagents are well known in the art and are not the subject of this invention. The labeled entity is impregnated into the first zone using the lyophilizing process described above.

The second zone contains an antigen-antibody pair partner of the labeled entity impregnated into the first zone. In the preferred embodiment, the labeled entity in the first zone is an antibody, therefore, the antigen-antibody pair partner in the second zone is an antigen which specifically binds to the antibody. The antigen-antibody pair partner is immobilized and contained within the second zone so that it cannot diffuse out. It may be bound directly to the second zone matrix or bound to another molecule or particule which is restrained from leaving the second zone.

The trapping layer containing bound antigen or bound antibody can be formed in such a manner that the trapping reagent remains immobilized in the second zone, keeping the trapped reagent and label in the trapping zone through immobilization. The trapping zone can be made of materials that through their chemical nature act as an immobilizing site for antigen or antibody. An example of such material is nitrocellulose or diazobenzyloxymethyl (DBM) paper. Nitrocellulose paper directly binds proteins and has been shown to be useful for immobilizing antigens. DBM matrix can bind DNA, RNA and proteins by means of covalent linkages through a diazonium group. Additionally other materials can be used which can immobilize the antigen or antibody through either physical or chemical means. For example, conventional biotechnology substrates such as polyacrylamide, gelatin, agarose, collagen and many others. The trapping reagent can be trapped within the material using chemical crosslinking agents that can react with a functional group in the antigen or antibody trapping reagent and with a functional group in the support material. The trapping reagents can be combined with a support that as a result of physical bonding, such as hydrophobic-hydrophobic bonding, can tend to trap the antigen within the zone. Further, the size of the antigens can contribute to its immobilization within the zone. For example larger antigens such as cells, viruses, cell organals and other cell subunits can be trapped in a porous matrix having a pore size smaller than the organelle or cell unit. Lastly, the trapping reagent can be covalently bound to a particle having a particle size greater than the matrix forming the base of the second layer.

The third zone is removed from the first and second zones. It contains elements of the detection means which will respond to the presence of the labeled entity originally impregnated in the first zone, which may be conveyed to the third zone, which may be conveyed to the third zone through the second zone by the test fluid when the test fluid contains an amount of the analyte. In some embodiments, there can be means to prevent certain of the elements from leaving the third zone to enter either of the other zones where they may interact with the labeled entity before it enters the third zone, thereby allowing a false indication of the positive presence of the analyte in the test fluid.

Suitable detection means are well known in the art. The detection means must coordinate with and be appropriate for the label which is used. The label is attached to the antigen-antibody pair partner impregnated into the first zone. In preferred embodiments, the label is covalently linked to this antigen or antibody. Methods used to conjugate or covalently crosslink various labels to antigens or antibodies are well known in the art.

When the label is present in the third zone along with elements of the detection means, the label characteristically stimulates a response by the detection means.

In the preferred embodiments, the label is an enzyme as discussed hereinabove. Although the present invention is not limited to these enzyme labels, the enzymes cited hereinabove each have a variety of detection means which may be appropriate for that enzyme in various formats. These detection means and formats are well known in the art. For example, an enzyme label may react directly with a color-forming reagent which becomes colored when it has reacted with the enzyme, or the enzyme may react with a substrate whose reaction product or products interact with a color-forming reagent to give a color. Many indirect means of having a great variety of forms exist.

Suitable substrates or color-forming reagents are well known in the art. In this regard, a number of different types of purified enzymes are commonly labeled reagent for use in immunoassays such as ELISA's. These include horseradish peroxidase, alkaline phosphatase, glucose oxidase, and beta-galactosidase. The present invention is not limited to the use of these enzymes as labels, however. Substrates well known in the art include substrates such as diaminobenzidine or p-nitrophenylphosphate react with horseradish peroxidase in the presence of hydrogen peroxide to form a brown to black color. In one form of the present invention hydrogen peroxide can be added exogeneously during the use of the device. Alternatively, the third zone can contain bound glucose oxidase and the first or second zone can contain glucose. Upon use of the device, the glucose in the first zone diffuses into the third zone and generates hydrogen peroxide by reacting with glucose oxidase.

An enzyme linked to an antigen-antibody pair partner can produce a color in the third zone by an indirect means such as effecting the permeability of the zone. An example of this method found suitable for the present invention is the use of highly purified collagenase as the enzyme label. This enzyme degrades collagen into small peptides. To detect the collagenase label two reagents which combine to form a color are separated by a collagen matrix barrier. The presence of the collagenase label is detected because the collagenase degrades the collagen barrier and allows the separated reagents to intermix and form a color. A suitable collagenase is that obtained from clostridium histolyticum purified by means well known in the art. A suitable collagen matrix is that formed by bovin type I collagen in triple helical (native) or denatured (gelatin) form.

In preferred embodiments, the test device comprises a carrier strip to support the enclosure and the matrices comprising the three zones. The carrier may be made out of any rigid, durable material. In the most preferred embodiments, the carrier is incorporated into the enclosure and becomes part of the enclosure structure as is the case in the structure schematically illustrated in Fig. 4. In such cases the strip material must be made of a material which will not react with any of the reagents or other chemical elements of the test, or carry materials that will diffuse into the test fluid or the matrix. Any polymeric or hard plastic material with the aforementioned properties will be suitable. In Fig. 4, the carrier has a matrix aperture in which the matrix or a zone such as the third zone of the matrix is enclosed and supported by a single-sided adhesive polystyrene film which seals the hole and form a portion of the enclosure.


Description of the Drawings

FIGURE 1 is an illustration of an antigen test strip having the preferred three-zone matrix 10 of an enzyme labeled antibody immobilized antigen embodiment of the present invention. The matrix 10 is constructed in three distinct layers or zones 12, 14, and 16 whose edges are sealed by sealing layer 11. Layer 12 forms the first zone, a labeled zone, layer 14 forms the second zone, a trapping zone, and layer 16 forms the third zone, a detection zone. The matrix 10 is shown positioned on a supporting member 20. Layer 12 is made of a porous material which contains soluble enzyme-linked antibody reagent 22. Layer 14 is also made of a porous material and contains immobilized antigen 24 that can trap unbound antibody. Layer 16, likewise, is made of a porous material and contains a color-forming reagent 26 that can detect the antibody label. In the presence of the enzyme-linked antibody 22, the color-forming reagent 26 will react to produce a detectable color reaction.

FIGURE 2 is an illustration showing the method of the invention using the device of Fig. 1. Specifically, a test fluid, generally identified by 28, is applied to the surface 30 of the first zone 12. As the test fluid 28 diffuses through the first zone 12, the fluid is held within the matrix by sealing layer 11. Within the matrix, free antigen 32 contacts and combines with the enzyme-linked antibody 22. After a short incubation period, the enzyme-linked antibody 22 with saturated recognition sites freely diffuses through the second zone 14 into the third zone 16 where the enzyme label on the enzyme-linked antibody 22 reacts in cooperation with the color-forming reagent 26 to produce a distinctive color reaction which indicates the presence of free antigen in the test fluid. Sealing layer 11 prevents bypass of the second zone by enzyme labeled antibody 22 into the third zone. Bypass of the second zone by enzyme-linked antibody having no bound antigens can result in a false signal.

FIGURE 3 is a diagrammatic illustration showing the method of the invention using the device of Fig. 1 and shows application of test fluid devoid of antigen. Specifically, a test fluid, generally designated 34, is applied to the surface 30 of the first zone 12. As the test fluid 34 diffuses through the first zone 12, the enzyme-linked antibody 22 is carried into layer 14 where the antibody binding sites recognize and bind to immobilized antigen 24. Accordingly, no enzyme-linked antibody can diffuse into the third zone 16 and no color change is observed, indicating that no free antigen was present in the test fluid. In the absence of sealing layer 11, the enzyme labeled antibody 22 could bypass the second zone 14 and generate a positive signal in a test of a negative test fluid.

FIGURE 4 shows a schematic illustration of a cross-section of a preferred embodiment of the present invention. The matrix comprises the first zone 12, the second zone 14, and the third zone 16 in sequential order. The first zone 12 is supported by the second zone 14. The second zone is supported by a two-sided adhesive coated strip 29, and by the third zone 16. The third zone 16 sits in an opening 20 in a plastic carrier 21 which supports the two-sided adhesive coated strip 29. The third zone is supported by a single-sided adhesive coated strip 23 whose peripheral edge is sealed to the plastic carrier 21. The matrix is enclosed within an enclosure comprising a single-sided adhesive coated film 25 whose peripheral edges are sealed to the plastic carrier 21, the body of the plastic carrier 21 and the single-sided adhesive coated film or strip 23 which seals the bottom of the opening 20 in the plastic carrier 21. The enclosure, film 25, has a first opening 27 adjacent to the first zone 12 for test fluid application. The peripheral edge of the second zone 14 is sealingly engaged to the double-sided adhesive coated strip 29 which is adjacent to and sealingly engaged to the plastic carrier 21. The enclosure has a second opening 31 in the single-sided adhesive coated strip 23 adjacent to the opening 20 in the plastic carrier 21 to allow the escape of displaced air.

FIGURE 5 shows an isometric exploded view of a preferred embodiment of the test strip of the present invention. The Liotta matrix 51, 52, 53 is mounted on plastic carrier strip 54 which bears a layer of pressure sensitive adhesive 57. In assembling the test device, the carrier strip 54 is sealed with an adhesive coated strip or film 56. An aperture 58 in the carrier strip 54 provides a location for the third zone 53 which fits within the aperture on the plastic strip 56. When assembly is complete the fluid zone 53 is protected from environmental variables by the sealing film 56. The second zone 52 is applied to the plastic carrier 54 and sealingly engaged thereto through the adhesive layer 57. The first zone 51 is in turn applied to the second zone 52 and then is in turn covered and sealed by the adhesive coated film 55 completing the assembly of the test device. Aperture 58 is supplied in the film 55 for the controlled application of test fluid. Aperture 59 is supplied in the film 56 to permit the escape of displaced air during the diffusion of the test fluid through zones 51, 52, and 53.

The following specific Examples further illustrate the practice of the present invention and include a best mode.

### Example I

### Three Layer Embodiment of the Invention for Detection of Theophylline

Materials:

   a. Antigen: Bovine Serum Albumin (BSA) with a theophylline hapten (BSA-theophylline).
   b. Antibody: Rabbit antibody to theophylline.
   c. Enzyme linked to antibody: horseradish peroxidase (HRP).
   d. Color forming enzyme substrate: tetramethyl benzidine (TMB).
   e. Peroxide generating system: $\beta$-D-glucose, glucose oxidase.
   f. Matrices forming layers:
   layers 1 and 3: cellulose;
   layer 2: derivitized nylon.

Procedure:

Step 1:

Preparation of layer containing color forming reagent: Cellulose sheets were cut into squares 6cm $\times$ 6cm. These sheets were dipped for 30 seconds in a solution containing glucose oxidase, 200 U/ml; in citrate buffer (0.1 M $NaH_2PO_4$, 0.05M citric acid, pH 5.5); and were dried in an oven at 40° for 30 minutes. These dried sheets were then dipped in a second solution containing tetramethyl benzidine 2mg/ml in methanol, and were dried in an oven at 100° C. for 1 minute.

Step 2:

Preparation of antigen coated layer: Derivitized nylon sheets were cut into squares of 6cm $\times$ 6cm and were then dipped and soaked for 24 hours in a solution containing 1mg/ml of BsA-theophylline in a phosphate buffered saline at pH 7.2. They were then removed and dried in an oven at 40° C. for 10 minutes. They were then removed and washed in a series of three baths of PBS, pH 7.2. They were then dried at 40° C. for 10 minutes.

Step 3:

Preparation of enzyme-linked antibody containing layer: Enzyme-linked antibody was prepared as described previously. This was then solubilized at a concentration of 5μg/ml in a Tris buffer (0.1M) at pH 7.2 containing 2% w/v ovalbumin and 0.5% v/v Triton-X-100 detergent and 15 mg/ml glucose. Cellulose sheets were made at 6cm × 6cm square and dipped into this solution for 30 seconds. They were then dried at 30° C. for 45 minutes.

Step 4:

Preparation of three-layer test device: Opaque polystyrene was obtained in roll stock on a 3 inch core. A 12″ segment was cut from this stock and a series of 9/16″ square holes were punched through at intervals of 3/4″ on center. To the back side of the polystyrene was laminated a transparent tape, of the acrylic adhesive type, that covered the holes and formed windows. Two pinholes were punched into each window to act as vents. Both layer 1 (antibody-enzyme layer) and layer 3 (color-forming) were cut into 1/4″ squares. Layer 3 was then placed onto the sticky side of each tape window. A separate piece of transparent adhesive tape was punched with a series of round holes of 1/8″ diameter at intervals of 3/4″ on center. Centered onto each of these holes on the sticky side was a 1/4″ square of layer 1. Layer 2 (antigen layer) was cut into 1cm × 1cm squares, and were centered over the layer 1 on the transparent adhesive such that layer 1 was completely covered. The layer 2 was then adhered peripherally to the tape. The layer 1 and 2 assembly was then adhered to the layer 3 assembly by centering each layer 1-layer 2 combination over a layer 3 and adhering the tops to the polystyrene. The tests were then cut into individual tests, each 3/4″ wide.

The completed device can be described as a single test dipstick of dimensions 3/4″ by 3″ by 1/8″ with a transparent viewing window on the back side and a sample application port on the top side.

Step 5:

To perform the assay, 40 milliliters of serum containing previously assayed levels of theophylline ranging from zero to 24μg/ml of theophylline. The reaction response was monitored by determining the reflectance of each trial after two minutes by a hand-held reflectance instrument. The color reflectance is directly proportional to the intensity of the color produced. The results can be viewed in Table 1.

## Table 1

| Trial Number | μg/ml Theophylline in Sample | Average of 4 Replicates (Reflectance) |
|---|---|---|
| 1 | 0 | .33 |
| 2 | 2.6 | .38 |
| 3 | 5.2 | .40 |
| 4 | 10.4 | .43 |
| 5 | 15.5 | .48 |
| 6 | 19.4 | .55 |
| 7 | 24.3 | .64 |

Example II

Three Layer Embodiment of the Invention for Pregnancy Detection

Materials:

    a. Antigen: human chorionic gonadotropin (hCG).
    b. Antibody: mouse antibodies to human (hCG).
    c. Enzyme linked to antibody: horseradish peroxidase.
    d. Color forming enzyme substrate: tetramethyl benzidine.
    e. Matrix material forming layers:
layers 1 and 3: cellulose;
layer 2: derivitized nylon.

Procedure:

Step 1:

Preparation of layer containing color forming reagent is as described for the theophylline test with the following changes: Tetramethyl benzidine was present at 4 milligrams/ml and the first dip was in 0.5M MES buffer, pH 6.0.

Step 2:

Preparation of antigen containing layer is as described for the theophylline test with the following changes: hCG as the coating antigen was present in solution at 2000 mIU/ml in 0.5M potassium phosphate buffer, pH 7.0.

Step 3:

Preparation of enzyme-linked antibody containing layer is as described for the theophylline test with the following changes: The enzyme-linked antibody was prepared using a maleimide coupling mechanism such as that discussed by Ishikawa et al (Journal of Immunoassay, Vol. 4, No. 3, 1983). This was incorporated at a concentration of 5-20 nanograms/test and dried at 40° C. for 7.5 minutes.

Step 4:

The test strip was assembled as described for the theophylline test.

Step 5:

Urine containing assayed quantities of hCG at 0 mIU and 200 mIU were prepared and added to the test strip in volumes of 25-35 µl. At the end of two minutes from sample application, the result was recorded by photocopy and documented. A blue color constitutes a positive test and no color constitutes a negative test. See Table II for results.

<u>Table 2</u>·

| <u>Sample Result</u> | <u>0 mIU hCG</u> | <u>200 mIU hCG</u> |
|---|---|---|
| | Negative (no color development) | Positive (color change noted) |

## Claims

1. A test device for determining the presence of an analyte-antigen in a test fluid, which comprises a matrix within an enclosure having a first opening for application of the test fluid, said matrix comprising (a) a first zone containing a labeled-antibody composition having at least one specific analyte-antigen binding site; (b) a second zone containing immobilized antigen composition that can bind specifically with an analyte-antigen binding site on said labeled-antibody; and (c) a third zone, removed from said first and second zones, containing detection means for indicating the presence of the label of said labeled-antibody within said third zone; wherein said test device comprises means for preventing bypass of the second zone by test fluid conveying labeled-antibody from the first zone to the third zone, and wherein any analyte-antigen composition present in said test fluid binds to said labeled-antibody composition protecting said labeled-antibody from reaction with said immobilized antigen in the second zone.

2. A test device as claimed in claim 1, wherein means for preventing bypass of the second zone by test fluid comprises an arrangement of the first zone, the second zone and the third zone such that fluid communication between the first zone and the third zone is exclusively provided by the second zone.

3. A test device as claimed in claim 1, wherein means for preventing bypass of said second zone comprises the enclosure sealingly engaging at least said second zone to eliminate bypass of said second zone by test fluid conveying labeled-antibody.

4. A test device as claimed in claim 3, wherein each of the first, second and third zones comprises a sheet-like layer having a peripheral edge, said second zone being positioned between the first zone and the third zone, wherein the edge of the second zone layer outwardly extends past the edge of the first zone and the edge of the third zone.

5. A test device as claimed in any of claims 1 to 4, wherein said enclosure further comprises at least one second opening to facilitate passage of test fluid through the matrix by permitting escape of displaced air from the enclosure.

6. A test device as claimed in claim 5, wherein said enclosure reproducibly fixes the volume of test fluid which may be effectively applied to the matrix through said first opening.

7. A test device as claimed in claim 5 or 6, wherein said enclosure further comprises additional openings to facilitate passage of test fluid through the matrix by permitting escape of displaced air from the enclosure.

8. A test device as claimed in claim 2, wherein said means for preventing bypass comprises a carrier strip in conjunction with said second zone of said matrix, wherein said carrier strip comprises an opening effective for the passage of test fluid from the second zone to the third zone.

9. A test device as claimed in claim 8, wherein exclusive fluid communication between the first zone and the third zone is provided by a second zone having a peripheral edge bound to the carrier strip to effect a seal between the strip and the peripheral edge of the second zone.

10. A test device as claimed in any of claims 1 to 9, wherein the binding capacity of, or amount of, immobilized antigen in the second zone exceeds the amount of labeled-antibody in the first zone.

11. A test device as claimed in claim 10, wherein the binding capacity of immobilized antigen contained within second zone exceeds the amount of labeled-antibody in the first zone by a ratio of at least about 1.5 to greater than 1 binding sites in the second zone per each binding site on the labeled-antibody.

12. A test device as claimed in any of claims 1 to 11, wherein the magnitude of the signal resulting from the presence of said label in the third zone is proportional to the concentration of analyte-antigen in the test fluid.

13. A test device as claimed in any of claims 1 to 12, wherein the labeled antibody composition has more than one specific analyte-antigen binding site.

14. A test device as claimed in any of claims 1 to 13, wherein said label of said labeled-antibody comprise an enzyme.

15. A test device as claimed in claim 14, wherein said enzyme is horseradish peroxidase, alkaline phosphatase, or glucose oxidase.

16. A test device as claimed in any of claims 1 to 15, wherein the analyte-antigen is theophylline, digoxin, an antigen of Streptococcus A, tetrahydrocannabinol, secobarbital, human chorionic gonadotropin or leutinizing hormone.

17. A test device as claimed in claim 16, wherein the antigen of Streptococcus A comprises a bacterial cell wall sub-unit of Streptococcus A or the Streptococcus A microorganism.

18. A test device for determining the presence of an analyte-antigen in a test fluid, which device comprises (a) a carrier strip with an aperture having an edge; (b) a matrix comprising (i) a first zone containing a labeled-antibody composition having at least one specific analyte-antigen binding site, (ii) a second zone containing immobilized antigen composition that can bind specifically with said analyte-antigen binding site on said labeled-antibody, and (iii) a third zone removed from said first and second zones containing detection means for indicating the presence of the label of said labeled-antibody within said third zone, wherein said first, second and third zones comprise sheet-like zones having a peripheral edge, the peripheral edge of the second zone extending outwardly past the edge of the first zone and the edge of the second zone, wherein said third zone is contained in the carrier strip aperture, and wherein the peripheral edge of the second zone is adhesively sealed to the edge of the aperture of the carrier strip; and (c) a clear film sealingly adhered to the carrier strip enclosing the matrix.

19. A test device as claimed in claim 18 wherein the test strip has a third zone side, said third zone held within the carrier strip aperture, and a second zone side having the second zone adherently bonded, wherein the second zone side is enclosed by a clear film sealingly adhered to the carrier strip and wherein the third zone is enclosed within a second clear film sealingly adhered to the carrier strip, fully enclosing the matrix.

20. A test device as claimed in claims 18 or 19 wherein the clear film has a first opening for application of test fluid and a second opening to facilitate passage of test fluid through the matrix by permitting escape of air displaced from the enclosed matrix.

21. A test device as claimed in any of claims 18 or 20 wherein the binding capacity of the immobilized antigen in the second zone exceeds the amount of labeled antibody in the first zone.

22. A test device as claimed in any of claims 18 to 21 wherein the magnitude of the signal resulting from the presence of the label in the third zone is proportional to the concentration of the analyte-antigen in the test fluid.

23. A test device as claimed in any of claims 18 to 22 wherein said label of said labeled antibody comprises an enzyme.

24. A test device as claimed in claim 23 wherein said enzyme is horseradish peroxidase, alkaline phosphatase or glucose oxidase.

25. A test device as claimed in any of claims 18 to 24 wherein said analyte-antigen is theophylline, digoxin, Streptococcus A, tetrahydrocannabinol, secobarbital, human chorionic gonadotropin or leutinizing hormone.

26. A test device as claimed in any of claims 18 to 25 wherein said labeled antibody composition has more than one specific analyte-antigen binding site.

27. A method of using a test device for determining the presence of an analyte-antigen in a test fluid, which comprises using a device as claimed in claims 1 to 26, resulting in the generation of a signal in said third zone.

FIG. 1

FIG. 2

INCUBATION

FIG. 3

INCUBATION

## FIG. 4

## FIG. 5